# EUROPÄISCHE PATENTSCHRIFT

(11) **EP 3 304 207 B1**
(45) Veröffentlichungstag und Bekanntmachung des Hinweises auf die Patenterteilung: **03.07.2019**
(21) Anmeldenummer: 16728860.4
(22) Anmeldetag: 26.05.2016
(51) Int. Cl.: G03F 7/20, G03F 7/00, B29C 39/14, C12N 5/00, C12N 5/0775, C12M 1/12, B82Y 40/00, G03F 1/70, G03F 1/00

(54) **NACHBILDUNG EINER STAMMZELLNISCHE EINES ORGANISMUS SOWIE VERFAHREN ZU DEREN ERZEUGUNG**
REPRODUCTION OF A STEM CELL NICHE OF AN ORGANISM AND METHOD FOR THE GENERATION THEREOF
RÉPLICATION D'UNE NICHE DE CELLULES SOUCHES D'UN ORGANISME ET PROCÉDÉ DE PRODUCTION DE LADITE RÉPLICATION

(30) Priorität: 29.05.2015 DE 102015108566; 21.12.2015 DE 102015122375
(43) Veröffentlichungstag der Anmeldung: 11.04.2018
(73) Patentinhaber: Technische Universität Ilmenau, 98693 Ilmenau (DE); Universitätsklinikum Jena, 07743 Jena (DE)
(72) Erfinder: SCHOBER, Andreas, 99096 Erfurt (DE); HAMPL, Jörg, 99090 Kühnhausen (DE); WEISE, Frank, 99425 Weimar (DE); BOROWIEC, Justyna, 98693 Ilmenau (DE); FERNEKORN, Uta, 99094 Erfurt (DE); GEBINOGA, Michael, 98693 Ilmenau (DE); SINGH, Sukhdeep, 98693 Ilmenau (DE); SCHLINGLOFF, Gregor, 98693 Ilmenau (DE); HÄFNER, Sebastian, 01217 Dresden (DE); BECK, James, 07749 Jena (DE); MÜLLER, Angelika, 07747 Jena (DE); SCHAEFFER, Astrid, 07749 Jena (DE)
(74) Vertreter: Engel, Christoph Klaus
(86) Internationale Anmeldenummer: PCT/EP2016/061883
(87) Internationale Veröffentlichungsnummer: WO 2016/193107

(56) Entgegenhaltungen:
- EP-A2- 2 485 247
- US-A1- 2009 298 166
- US-A1- 2014 329 323
- US-A1- 2015 024 026
- US-A1- 2015 118 197

## Beschreibung

Die vorliegende Erfindung betrifft ein Verfahren zum Nachbilden einer Stammzellnische eines Organismus.

Ein Beispiel für die Aufnahme und Analyse von Stammzell-Morphologien gemäß dem Stand der Technik ist in dem wissenschaftlichen Artikel von Celso, C. L.; Fleming, H. E.; Wu, J. W. et al.: "Live animal tracking of individual stem/progenitor cells in their niche" in Nature, Ausgabe 457, Seiten 92-96, Januar 2009 gezeigt.

Der Einsatz von Hydrogelen zur Nachbildung von Stammzellnischen findet sich in dem Artikel von Gerecht, S. et al.: "Hyaluronic acid hydrogel for controlled self-renewal and differentiation of human embryonic stem cells" in PNAS, Ausgabe 104, Nr. 27, Seiten 11298-11303, Juli 2007.

Ein weiterer Ansatz stammt von Hashimoto, Y. et al.: "The effect of decellularized bone/bone marrow produced by highhydrostatic pressurization on the osteogenic differentiation of mesenchymal stem cells" in Biomaterials, Ausgabe 32, Seiten 7060-7067, 2011. Hier werden Knochen menschlichen Ursprungs eingesetzt.

Eine mikrotechnische Lösung sind Kapillarenarrays, wie sie in dem Artikel von Su, W.-T.: "Ex vivo expansion of a hematopoietic stem cell on a murine stromal cell by 3D micropillar device" in Biomed Microdevices, Ausgabe 13, Seiten 11-17, 2011, aufgeführt sind .

In dem Artikel von Burke, D. P. et al.: "Substrate stiffness and oxygen availability as regulators of mesenchymal stem sell differentiation within a mechanically loaded bone chamber" in Biomech Model Mechanobiol, Ausgabe 14, Seiten 93-105, 2015, sind Simulationen zum Einfluss mechanischer Faktoren bzw. der Oxigenierung gezeigt.

Der Artikel von Housler, G. J. et al.: "3-D Perfusion Bioreactor Process Optimization for CD34+ Hematopoietic Stem Cell Culture and Differentiation towards Red Blood Cell Lineage" in Journal of Bone Marrow Research, Ausgabe 2, Nr. 3, 2014, beschreibt einen bioreaktorbasierten Ansatz, bei dem jedoch Hohlfasern genutzt werden.

Der Artikel von Prendergast, Ä. M. et al.: "Hematopoietic stem cells, infection, and the niche" in Annals of the New York Academy of Sciences, Ausgabe 1310, Seiten 51-57, 2014 beschreibt die Infizierung von Stammzellen in der Stammzellnische.

Die US 2015/0118197 A1 zeigt ein Verfahren zur Herstellung eines elektrogesponnenen Gewebes, durch welches beispielsweise die Morphologie einer Nische in den Vogt-Palisaden eines Limbus nachgebildet werden kann.

Die Aufgabe der vorliegenden Erfindung besteht ausgehend vom Stand der Technik darin, verbesserte Kulturbedingungen für Stammzellen schaffen zu können.

Die genannte Aufgabe wird gelöst durch ein Verfahren gemäß dem beigefügten Anspruch 1.

Das erfindungsgemäße Verfahren dient zum Nachbilden mindestens einer Stammzellnische eines Organismus. Das erfindungsgemäße Verfahren dient insbesondere zum biolithomorphischen Nachbilden einer Stammzellnische eines Organismus. Bei dem Organismus handelt es sich bevorzugt um einen lebenden Organismus, in welchem die nachzubildende Stammzellnische ausgebildet ist. Bei dem lebenden Organismus handelt es sich insbesondere um ein Tier oder um einen Menschen.

Die Stammzellnische begrenzt einen Raum, in welchem eine Stammzelle des Gewebes des Organismus leben kann.

Durch das erfindungsgemäße Verfahren wird insbesondere die Morphologie der mindestens einen Stammzellnische nachgebildet. Daher ist die mindestens eine Stammzellnische im Sinne der Erfindung zumindest durch ihre geometrischen Eigenschaften definiert. Diese geometrischen Eigenschaften beschreiben insbesondere die räumliche Anordnung einer Wandung der Stammzellnische in mindestens zwei Dimensionen. Die Wandung der Stammzellnische umschließt den Raum, in welchem die Stammzelle des Gewebes des Organismus leben kann, nicht vollständig, damit beispielsweise ein Stoffaustausch der Stammzelle ermöglicht ist.

In einem Schritt des erfindungsgemäßen Verfahrens wird ein Abbild eines Gewebes des Organismus erzeugt. Das Gewebe, welches insbesondere durch Teile eines Organes oder durch ein vollständiges Organ gebildet sein kann, umfasst mindestens eine Stammzellnische. Zumindest diese eine Stammzellnische, aber bevorzugt mehrere der Stammzellnischen sollen erfindungsgemäß nachgebildet werden. Das Gewebe ist bevorzugt durch ein Gewebe eines Knochenmarks des Organismus gebildet. Das Abbild ist zumindest zweidimensional. In diesem Abbild sind u. a. die Wandungen der mindestens einen Stammzellnische abgebildet. Zumeist sind aber auch die Stammzellen und andere Strukturen des Gewebes abgebildet. Das Abbild ist bevorzugt durch eine Vielzahl an Bildpunkten gebildet, wobei jeder Bildpunkt zumindest eine Helligkeitsinformation oder eine Farbinformation besitzt. Das Abbild der der mindestens einen Stammzellnische wird erzeugt, indem ein abbildendes Verfahren auf das Gewebe angewendet wird. Das Abbild wird vor der weiteren Verarbeitung bevorzugt gespeichert.

In einem weiteren Schritt des erfindungsgemäßen Verfahrens wird das Abbild mithilfe von Methoden der Bildverarbeitung gefiltert, um ein Strukturbild der mindestens einen abgebildeten Stammzellnische zu erhalten. Im Strukturbild sind jedenfalls die Wandungen der mindestens einen Stammzellnische abgebildet. Bevorzugt sind im Strukturbild ausschließlich die Wandungen der mindestens einen Stammzellnische abgebildet. Das Abbild ist bevorzugt durch eine Vielzahl an Bildpunkten gebildet, wobei jedem Bildpunkt bevorzugt eine logische Information von einem Bit zugeordnet ist, ob an diesem Ort eine Wandung gegeben ist oder nicht. Insoweit ist das Strukturbild bevorzugt durch ein Schwarz-Weiß-Bild gebildet. Den Bildpunkten können aber auch einzelne Werte eines eine Vielzahl an Werten umfassenden Wertebereiches zugeordnet sein, um beispielsweise Abstufungen zu repräsentieren. Das Strukturbild ist zumindest zweidimensional. Bei weiteren bevorzugten Ausführungsformen besitzt das Strukturbild 2,5 oder drei Dimensionen.

In einem weiteren Schritt des erfindungsgemäßen Verfahrens wird aus dem Strukturbild eine lithographische Maske erzeugt. Die lithographische Maske ist dazu ausgebildet, ein formendes Bearbeitungsverfahren zur Strukturierung unmittelbar oder mittelbar ausführen zu können, um gemäß dem Strukturbild zu formen; nämlich um die im Strukturbild gezeigten Wandungen der Stammzellnische durch das formende Bearbeitungsverfahren nachzubilden. Bei dem formenden Bearbeitungsverfahren handelt es sich bevorzugt um ein photolithographisches Verfahren, um ein Prägeverfahren, um ein nanoprägelithographisches Verfahren, um ein heißprägendes Verfahren, um ein thermoformendes Verfahren oder um eine Kombination mehrerer der genannten Verfahren. Entsprechend ist die lithographische Maske beispielsweise dazu ausgebildet, durchleuchtet zu werden oder durch deren Anwendung ein formendes Werkzeug zu erstellen, welches anschließend zum Formen bzw. Strukturieren genutzt wird.

In einem weiteren Schritt des erfindungsgemäßen Verfahrens wird ein Ausgangsmaterial eines Substrates strukturiert, d. h. geformt, wofür die lithographische Maske mittelbar oder unmittelbar angewendet wird und wodurch mittelbar oder unmittelbar ein strukturiertes Substrat erhalten wird, welches die Nachbildung der mindestens einen abgebildeten Stammzellnische darstellt. Das Strukturieren des Ausgangsmaterials erfolgt mit dem oben beschriebenen formenden Bearbeitungsverfahren. Ist das formende Bearbeitungsverfahren beispielsweise durch ein photolithographisches Verfahren gebildet, so wird die lithographische Maske unmittelbar auf das Ausgangsmaterial angewendet. Ist das formende Bearbeitungsverfahren beispielsweise durch ein nanoprägelithographisches Verfahren oder durch ein heißprägendes Verfahren gebildet, so wird die lithographische Maske mittelbar auf das Ausgangsmaterial angewendet, da mit der lithographischen Maske zunächst ein Werkzeug erstellt wird, welches dann unmittelbar auf das Ausgangsmaterial angewendet wird. Die Nachbildung gleicht der abgebildeten Stammzellnische in ihren geometrischen Eigenschaften, d. h. in ihrer räumlichen Ausdehnung, wobei diese Übereinstimmung in mindestens zwei Dimensionen gegeben ist. Die Nachbildung ist jedoch grundsätzlich dreidimensional ausgebildet, sodass bei einer Übereinstimmung in zwei Dimensionen von einer 2,5-dimensional strukturierten Nachbildung gesprochen werden kann. Insbesondere weist die Nachbildung eine Wandung auf, die in ihrer Ausdehnung der originalen Wandung der abgebildeten Stammzellnische zumindest in zwei Dimensionen gleicht. Die Nachbildung ist bevorzugt mindestens 10 mm lang und 10 mm breit. Die Nachbildung ist bevorzugt mindestens 0,5 mm hoch.

Die resultierende Nachbildung der mindestens einen abgebildeten Stammzellnische ist als eine biolithomorphische Nachbildung anzusehen. Biolithomorphie ist die Übertragung der Fertigungsprinzipien der Mikro- und Nanotechnologien für die Konstruktion von biologischen dreidimensionalen Geweben für Anwendungen in den "Life Sciences". Dabei sind biologische Morphologien multiskalig auf 2D- und 3D-Substrate zur Zellkultivierung zu übertragen.

Bei bevorzugten Ausführungsformen der Erfindung wird das Gewebe dem Organismus durch Biopsie entnommen. Es werden bevorzugt Schnitte des entnommenen Gewebes angefertigt, die bevorzugt mit verschiedenen Farben eingefärbt werden, um Strukturen hervorzuheben, insbesondere um die mindestens eine abzubildende Stammzellnische hervorzuheben. Es werden bevorzugt die Wandungen der mindestens einen abzubildenden Stammzellnische hervorgehoben.

Bei bevorzugten Ausführungsformen der Erfindung umfasst das Strukturbild ein Teilstrukturbild für grobe Strukturen der abgebildeten Stammzellnische und ein Teilstrukturbild für feine Strukturen der abgebildeten Stammzellnische. Die lithographische Maske umfasst dabei eine Teilmaske für grobe Strukturen, die aus dem Teilstrukturbild für grobe Strukturen erzeugt wird. Entsprechend umfasst die lithographische Maske weiterhin eine Teilmaske für feine Strukturen, die aus dem Teilstrukturbild für feine Strukturen erzeugt wird.

Die feinen Strukturen weisen eine maximale Elementgröße auf, die bevorzugt zwischen 10 µm und 200 µm, besonders bevorzugt zwischen 50 µm und 75 µm beträgt. Die maximale Elementgröße beträgt bevorzugt 50 µm oder alternativ 75 µm. Die feinen Strukturen umfassen ausschließlich Strukturelemente, deren Ausdehnung höchstens so groß wie die maximale Elementgröße ist.

Die groben Strukturen weisen eine minimale Elementgröße auf, die bevorzugt zwischen 10 µm und 200 µm, besonders bevorzugt zwischen 50 µm und 75 µm beträgt. Die minimale Elementgröße beträgt bevorzugt 50 µm oder alternativ 75 µm. Die groben Strukturen umfassen ausschließlich Strukturelemente, deren Ausdehnung mindestens so groß wie die minimale Elementgröße ist.

Das Filtern des Abbildes umfasst bevorzugt verschiedene Kantenanalysen, mit denen das Teilstrukturbild für die groben Strukturen und das Teilstrukturbild für die feinen Strukturen bestimmt werden. Zum Bestimmen des Teilstrukturbildes für die groben Strukturen wird bevorzugt ein Tiefpass angewendet. Zum Bestimmen des Teilstrukturbildes für die feinen Strukturen wird bevorzugt ein Hochpass angewendet.

Bei bevorzugten Ausführungsformen der Erfindung wird mit der lithographischen Maske zunächst ein Werkzeug zum Verformen des Ausgangsmaterials des Substrates erstellt, wobei anschließend das Ausgangsmaterial des Substrates mit dem Werkzeug strukturiert wird.

Das Werkzeug zum Verformen des Ausgangsmaterials umfasst bevorzugt ein Werkzeug zur Erzeugung grober Strukturen und ein Werkzeug zur Erzeugung feiner Strukturen, wobei das Werkzeug zur Erzeugung grober Strukturen mit der Teilmaske für grobe Strukturen erstellt wird, und wobei das Werkzeug zur Erzeugung feiner Strukturen mit der Teilmaske für feine Strukturen erstellt wird.

Das Werkzeug zur Erzeugung feiner Strukturen ist bevorzugt durch ein Prägewerkzeug zum Heißprägen, durch ein Prägewerkzeug für eine Nanoprägelithographie, durch eine Form zum Abgießen oder durch eine Form zum Spritzprägen gebildet. Das Werkzeug zur Erzeugung grober Strukturen ist bevorzugt durch ein Thermoformwerkzeug gebildet.

Das Ausgangsmaterial ist bevorzugt durch eine Folie gebildet. Die Folie ist bevorzugt porös. Das Ausgangsmaterial ist alternativ durch ein Polymer gebildet, welches durch thermoplastische Verformung verformt und strukturiert wird.

Das Ausgangsmaterial bzw. das Substrat wird bevorzugt mit Bestandteilen einer extrazellulären Matrix der nachzubildenden Stammzellnische beschichtet.

Das strukturierte Substrat wird bevorzugt mit mindestens einer Stammzelle besiedelt. Die mindestens eine Stammzelle wird im strukturierten Substrat kultiviert.

Besonders bevorzugt wird ein Schritt durchgeführt, bei welchem die mindestens eine Stammzelle im strukturierten Substrat expandiert. Anschließend wird die mindestens eine expandierte Stammzelle bevorzugt aus dem strukturierten Substrat entnommen und in den individuellen Organismus, dessen Gewebe abgebildet wurde, eingeführt. Insoweit kann diese Ausführungsform der Erfindung auch ein Verfahren zum Expandieren von Stammzellen darstellen.

Bei einer alternativ bevorzugten Ausführungsform wird ein Schritt durchgeführt, bei welchem die mindestens eine Stammzelle im strukturierten Substrat zu einer Blutzelle ausdifferenziert. Die mindestens eine Blutzelle wird bevorzugt aus dem strukturierten Substrat entnommen und in den individuellen Organismus, dessen Gewebe abgebildet wurde, eingeführt. Insoweit kann diese Ausführungsform der Erfindung auch ein Verfahren zum Ausdifferenzieren von Stammzellen darstellen.

Die vorliegende Erfindung dient zur organnahen und bevorzugt organgetreuen geometrischen und bevorzugt auch biochemischen Replikation mindestens einer Stammzellnische, die bevorzugt hämatopoetisch ist. Durch die Erfindung kann einer Blutstammzelle in situ eine organnahe geometrische Umgebung geboten werden, die es ermöglicht, diese Blutstammzellen für therapeutische Zwecke gezielt zu expandieren. Durch die Erfindung kann eine hohe Expansionsrate erzielt werden. Ohne eine solche Umgebung neigen die Stammzellen zur Differenzierung und sind für den beabsichtigten therapeutischen Zweck nicht mehr einsetzbar.

Erfindungsgemäß dient das Abbild eines Gewebes eines Organismus, insbesondere präparierte Schnittbilder vom Knochenmark gesunder Patienten, als Ausgangsmaterial und Vorlagen. Diese werden über Bildverarbeitungstechniken (BV-Techniken) gefiltert und in die lithographische Maske übersetzt und bevorzugt durch Mikrostrukturtechnik abgebildet. Die so bevorzugt entstandene Siliziumstruktur wird bevorzugt über einen Abguss oder über eine galvanische Abformung in das polymere bzw. metallische Werkzeug überführt. Dieses dient dann bevorzugt der weiteren Replikation in dickwandige abgegossene oder in dünnwandige folienbasierte Kultivierungsstrukturen, die als Scaffolds bezeichnet werden können und durch strukturierte Substrate gebildet sind.

Bei der erfindungsgemäßen Erzeugung des strukturierten Substrates in einer bevorzugten Form eines folienbasierten Scaffolds werden bevorzugt zwei Verfahrensweisen angewendet, um eine sehr hohe Strukturierungstiefe, ähnlich dem nativen Knochenmark, zu erhalten. Eine Foliendicke liegt hier bevorzugt bei max. 75 µm. In einem zweigeteilten BV-Prozess können aus einem Knochenmarkschnitt zuerst verschiedene Strukturgrößen isoliert werden. Dabei wird bevorzugt mit einem Hochpass- und einem Tiefpassfilter gearbeitet. Große Strukturen werden dabei gesondert in die eigene lithografische Maske für grobe Strukturen überführt; ebenso verhält es sich mit kleinen Strukturen. Die Grenze liegt bevorzugt bei 50 µm bis 75 µm Elementgröße. Aus der Maske für die kleinen Strukturen wird ein Prägewerkzeug hergestellt. Dieses Prägewerkzeug besteht bevorzugt aus Metall, Silizium oder einem sehr steifen organischen Material (Photoresist/X-PDMS). Mit diesem Prägewerkzeug wird bevorzugt mittels Heißprägen oder mithilfe der Nanoprägelithografie eine Feinstruktur auf die Folie aufgeprägt, wodurch sie eine Mikrostruktur erhält. Aus der Maske der großen Strukturen im Knochenmarkschnitt wird bevorzugt ein Thermoformwerkzeug hergestellt. Auch hierbei können für das Werkzeug verschiedenen Materialen eingesetzt werden. Die texturierte Folie wird nun in eine Thermoformanlage eingelegt und die Grobstruktur in die Folie eingeformt, sodass sie eine Mesostruktur erhält. Resultat ist das in zunächst zwei Größenordnungen strukturierte Substrat. Dieses kann durch Verwendung eines geeigneten Oberflächenbehandlungsprozesses auch noch bevorzugt nanostrukturiert werden, sodass es eine Nanostruktur erhält. Beispiele hierfür können chemische Ätzung, Plasmamodifikation oder das Aufbringen von Hydrogel- oder anderen organischen Beschichtungen ggf. auch mit Funktionsgruppen sein. Nach erfolgter Oberflächenmodifikation liegt das Substrat mit der Meso-, der Mikro- und der Nanostruktur vor.

Die Verwendung einer porösen Folie als Halbzeug ermöglicht zudem auch die Herstellung eines durchströmbaren Scaffolds.

Nachfolgend sind weitere bevorzugte Ausführungsformen der Erfindung beschrieben.

Zum Erzeugen des Abbildes eines mindestens eine Stammzellnische umfassenden Gewebes eines Organismus werden bevorzugt Schnittbilder als das Abbild des Gewebes des lebenden Organismus hergestellt. Bei dem lebenden Organismus handelt es sich bevorzugt um ein Tier oder um einen Menschen. Hierfür wird bevorzugt zunächst Gewebe in Form von Knochenmark mittels Biopsie gewonnen. Es werden pathologische Mikroschnitte mit einer Schnittdicke von etwa 10 µm angefertigt und diese mit Hämatoxylin-Eosin gefärbt. Es erfolgt bevorzugt eine verschiedenartige Färbung von Knochenmarkstruktur, die weiches Gewebe, mindestens eine extrazelluläre Matrix, und eine Spongiosa-Struktur, d. h. harte Knochenstruktur umfasst.

In einem weiteren bevorzugt durchzuführenden Schritt erfolgt ein Digitalisieren der Schnittbilder in mikroskopische Aufnahmen. Bevorzugt erfolgt ein Digitalisieren der einzelnen verschiedenen Färbungen, sodass mehrere Bilder entstehen. Alternativ bevorzugt erfolgen zunächst ein Digitalisieren in einzelnes Bild und ein späteres Erkennen und Trennen der Strukturen durch Anwenden von Methoden der Bildverarbeitung. Bei der Bildverarbeitung erfolgt bevorzugt zunächst ein Erzeugen von Graustufenbildern aus den digitalisierten Schnittbildern. Es werden mindestens ein Hochpass und/oder ein Tiefpass auf die Graustufenbilder angewendet, um die Graustufenbilder in eine Grobstruktur und in eine Feinstruktur zu trennen. Bevorzugt erfolgt weiterhin eine Kantendetektion unter Nutzung von Kantenfindungsalgorithmen, wie z. B. der Canny- oder Niblack-Algorithmus, und/oder unter Nutzung einer Schwellwertbildung. Aus den detektierten Kanten wird bevorzugt jeweils ein Schwarz-Weiß Bild erstellt. Das mindestens eine Schwarz-Weiß-Bild wird bevorzugt vektorisiert.

Zum Erzeugen der lithographischen Maske wird das vektorisierte Bild der detektierten Kanten überführt. Bevorzugt wird das vektorisierte Bild der detektierten Kanten durch ein mehrfaches Anreihen, Spiegeln oder Rotatieren vergrößert.

In einem weiteren bevorzugt durchzuführenden Schritt wird das Werkzeug zur Abformung erstellt. Hierfür wird die lithographische Maske bevorzugt prozessiert, z. B. als Hellfeld oder Dunkelfeld, was in verschiedenen Materialsystemen, wie Silizium oder Glas und unter Nutzung verschiedener Technologien, wie z. B. nasschemische oder trockenchemische Technologien erfolgen kann. Bevorzugt erfolgt eine Beeinflussung von Ätztiefe, Strukturauflösung und Kantenverrundung. Das Prozessieren der lithographischen Maske führt beispielsweise zu einem prozessierten Wafer, der bevorzugt mit einer galvanischen Startschicht für eine nachfolgende galvanische Abformung oder mit einer Anti-Adhäsionsschicht für einen nachfolgenden Abguss oder für eine nachfolgende Abformung beschichtet wird. Das Werkzeug zur Abformung ist bevorzugt direkt durch die geätzten Strukturen, durch deren galvanische Abformungen oder durch deren Abgüsse, z. B. aus hartem Silikon oder einem Epoxid-Harz, gebildet.

Das strukturierte Substrat, welches auch als Kultursubstrat bezeichnet werden kann, wird durch Nutzung des Werkzeuges zur Abformung erhalten. Dabei erfolgt bevorzugt eine Replikation des Werkzeuges zur Abformung. Diese Replikation erfolgt bevorzugt durch ein Abgießen. Das mindestens eine Kultursubstrat wird durch einen direkten Abguss repliziert und dann zur Kultivierung verwendet. Das Kultursubstrat ist massiv und weist an der Oberfläche eine Strukturierung auf. Bevorzugt wird durch eine flächige Beschichtung oder durch eine Schattenmaske ergänzend eine Nanostruktur auf das Kultursubstrat aufgebracht, was bevorzugt durch eine physikalische Gasphasenabscheidung erfolgt. Die Replikation erfolgt alternativ bevorzugt durch eine einfache Abformung. Das Werkzeug zur Abformung wird genutzt, um Strukturen durch ein Heißprägen oder durch ein Thermoformen abzuformen. Es entstehen einfach strukturierte Substrate aus massiven Körpern beim Heißprägen bzw. aus dünnen Folien beim Thermoformen. Die Folien sind bevorzugt porös. In einem weiteren bevorzugt durchzuführenden Schritt wird auf das Substrat eine flächige Beschichtung oder mithilfe einer Schattenmaske eine Nanostruktur aufgebracht. Die Replikation erfolgt alternativ bevorzugt durch eine mehrfache Abformung. Bei dieser Ausführungsform werden bevorzugt Folien mit einer Ausgangsdicke von maximal 100 µm verwendet. Diese Folien sind bevorzugt porös. Zunächst wird die Feinstruktur durch Heißprägen in die Oberfläche der Folie eingeprägt, wofür das Werkzeug zur Erzeugung feiner Strukturen genutzt wird. Dabei beträgt ein Aspektverhältnis bevorzugt maximal 3. Die feinstrukturierte Folie wird mit ihrer unstrukturierten Seite in eine das Werkzeug zur Erzeugung grober Strukturen bildende Thermoformmaschine eingelegt und durch Aufheizen und durch einen Druckstoß grob strukturiert. In einem weiteren bevorzugt durchzuführenden Schritt wird durch eine flächige Beschichtung oder durch eine Schattenmaske noch eine Nanostruktur auf das strukturierte Substrat aufgebracht.

In einem weiteren bevorzugt durchzuführenden Schritt des erfindungsgemäßen Verfahrens erfolgt eine Kultivierung von Stammzellen in dem strukturierten Substrat. Das strukturierte Substrat stellt bevorzugt ein statisches System dar. Das strukturierte Substrat, insbesondere wenn es massiv ausgeführt ist, wird in ein einfaches Kulturgefäß eingebracht und mit der Stammzellkultur besiedelt. Dabei wird bevorzugt ein periodischer Medientausch durchgeführt. Das strukturierte Substrat stellt bevorzugt ein durchströmtes System dar. Das Substrat, insbesondere wenn es folienbasiert und porös ausgebildet ist, wird bevorzugt in einem Bioreaktorsystem perfundiert betrieben. Dazu werden Zellkulturen auf das Substrat aufgebracht und nach einer kurzen Adhärenzzeit wird das Substrat in das Bioreaktorsystem eingebracht und mit einer entsprechenden Flussmenge durchströmt. Der Medienaustausch kann hierbei periodisch, kontinuierlich oder periodisch partiell sein. Das Substrat, insbesondere wenn es folienbasiert, massiv und unporös ausgebildet ist, wird bevorzugt in einer Superfusion betrieben. Dazu werden Zellkulturen auf das Substrat aufgebracht und nach einer kurzen Adhärenzzeit wird das Substrat in das Bioreaktorsystem eingebracht und mit einer entsprechenden Flussmenge überströmt. Der Medienaustausch kann hierbei periodisch, kontinuierlich oder periodisch partiell sein.

Es ist schon lange bekannt, dass die dreidimensionale Struktur des Mikromilieus einer Stammzelle einen wesentlichen Faktor für die Regulation der Hämatopoese darstellt. So konnte gezeigt werden, dass eine Desintegration von Knochenmarkbröckchen nach einer Collagenasebehandlung zum vollständigen Erlöschen der Hämatopoese in der anschließenden Kultur führt, während aus intakten Bröckchen selbst ohne externe Wachstumsfaktorgabe schon nach kurzer Zeit Stroma mit hämatopoetischen Inseln auswächst. Ein Defekt nach Collagenasebehandlung lässt sich nur ansatzweise durch Wachstumsfaktoren (SCF, IL3, Epo) kompensieren. Überraschend ist daher, dass bereits eine 2,5D-artige Struktur in Form eines erfindungsgemäß strukturierten Substrates eine wesentlich erhöhte Vitalität und Expansion der Stammzellen ermöglicht.

Wie schon ausgeführt, können beim erfindungsgemäßen Verfahren unterschiedliche Materialien als Ausgangsmaterial für das Substrat eingesetzt werden. Sowohl organische wie auch anorganische Materialien können dabei über eine geeignete Kopplungschemie mit zellspezifischen Molekülen vorteilhaft beschichtet werden. Eine alternativ bevorzugte Ausführungsform umfasst ein verfahrenstechnisches Auftragen, z. B. über "spincoating" von zellverträglichen Materialien, vorzugsweise von Bestandteilen der extrazellulären Matrix, wie z. B. Kollagen. Das Auftragen erfolgt vorzugsweise im Falle polymerer Substrate auf das Ausgangsmaterial. Das Auftragen erfolgt alternativ bevorzugt durch Tauchverfahren, wobei das Auftragen auf das bereits geformte Substrat erfolgt. Besonders bevorzugt erfolgt eine chemische Modifikation des 3D- bzw. 2,5D-strukturierten Substrates mit Zelladhäsionsmolekülen. Die Zelladhäsionsmoleküle sind bevorzugt durch geeignete Peptidsequenzen, z. B. aus der Familie der RGD Motive gebildet.

Im lebenden Körper werden aus biologischen Phosphaten, wie z. B. Glucose-6-phosphat, in die umgebende Matrix kontinuierlich Phosphationen freigesetzt. Durch das gleichfalls vorliegende Calcium kommt es beim Überschreiten des Löslichkeitsprodukts zur Kristallisation von Calciumphosphat und Ausbildung einer Knochenstruktur. Erfindungsgemäß wird bevorzugt ein polymeres Ausgangsmaterial verwendet, welches auch solche kristallinen Materialien enthält, wie sie im Knochen vorliegen.

Bei einer weiteren bevorzugten Ausführungsform erfolgt eine Konfektionierung bzw. Synthese des vorzugsweisen polymeren Substrates für die Stammzellkultivierung derart, dass ein Folie aus einer Kollagenmatrix und Hydroxylapatit (Ca₅[OH|(PO₄)₃]) als Ausgangsmaterial bereitgestellt wird und dann der gezeigten Strukturierung unterworfen wird.

Bei weiteren bevorzugten Ausführungsformen wird ein synthetisches Material wie z. B. eine synthetische Nanokompositstruktur, die beispielsweise aus verkieselten Poly(N-isopropylacrylamide)/Hydroxyapatit besteht, als Ausgangsmaterial für das Substrat genutzt.

Die genannten konfektionierten Polymere werden bevorzugt wiederum einer chemischen Modifikation unterzogen; vorzugsweise durch Zellkontakt vermittelnde Peptide oder durch andere Substanzen.

Bei einer weiteren bevorzugten Ausführungsform können unterschiedliche Ladungen durch Beschichtungen des Ausgangsmaterials bzw. des Substrates mit vorzugsweise Apatitanalogen Kristallen oder mit Hyaluronsäure-Hydrogelen mit jeweils unterschiedlicher Dotierung erfolgen. Die Vernetzung kann über die Einführung bisfunktioneller organischer Moleküle erfolgen. Dadurch gelingt auch die Kopplung von VLA4/VCAM, beta1-Integrin, WNT, IL6, SCF und CXCL12.

Des Weiteren werden bevorzugt durch Modifikationen im Polymer des Ausgangsmaterials bzw. des Substrates Gradienten von biologischen Effektormolekülen vorgelegt.

Das erzeugte Substrat wird bei bevorzugten Ausführungsformen der Erfindung in einen Systemaufbau eingebracht, der unterschiedliche physiologische Bedingungen, vorzugsweise auch die des Knochenmarks, simulieren kann. So besteht auch neben den oben beschriebenen biochemischen Modifikationen des Substrates hinsichtlich des Ausgangsmaterials, der Oberflächen, der Wachstumsfaktoren, der Adhäsionsproteine etc. eine vorteilhafte Möglichkeit die Kulturbedingungen, wie normoxische und hypoxische Zustände, durch geeignete Klimatisierung bzw. Fluidik einzustellen bzw. Wachstumsfaktoren und chemische/biochemische Effektorstoffe zu applizieren.

Eine bevorzugte synthetische Nachbildung mindestens einer Stammzellnische eines Organismus weist geometrische Eigenschaften der mindestens einen Stammzellnische des Organismus auf. Die Nachbildung umfasst zumindest eine Wandung, welche einer Wandung der Stammzellnische des Organismus in mindestens zwei Dimensionen nachgebildet ist und dieser gleicht. Diese Nachbildung weist somit die Morphologie der Stammzellnische des Organismus auf. Diese Nachbildung weist bevorzugt eine Mesostruktur als eine grobe Struktur und eine Mikrostruktur als eine feine Struktur sowie weiterhin bevorzugt eine Nanostruktur als eine weitere feine Struktur auf. Die grobe Struktur und die feine Struktur weisen bevorzugt die im Zusammenhang mit dem erfindungsgemäßen Verfahren beschriebenen Merkmale auf. Im Übrigen weist die Nachbildung bevorzugt auch solche Merkmale auf, die im Zusammenhang mit dem erfindungsgemäßen Verfahren beschrieben sind.

Weitere Vorteile, Einzelheiten und Weiterbildungen der Erfindung ergeben sich aus der nachfolgenden Beschreibung bevorzugter Ausführungsformen der Erfindung, unter Bezugnahme auf die Zeichnungen. Es zeigen:
- Fig. 1:: ein erfindungsgemäß erstelltes strukturiertes Substrat in einer Querschnittsdarstellung;
- Fig. 2:: ein erfindungsgemäß erstelltes Originalbild eines Knochenmarksabschnittes und daraus erfindungsgemäß erzeugte Strukturbilder;
- Fig. 3:: einen Vergleich zwischen dem in Fig. 2 gezeigten Originalbild und einem erfindungsgemäß erstellten Strukturbild;
- Fig. 4:: ein Flussdiagramm einer erfindungsgemäßen Erzeugung lithographischer Masken;
- Fig. 5:: erfindungsgemäß feinstrukturierte Polycarbonatfolien;
- Fig. 6:: die in Fig. 5 gezeigten Polycarbonatfolien mit ergänzenden groben Strukturen;
- Fig. 7:: ein Flussdiagramm einer physikalischen Oberflächenmodifikation gemäß einer bevorzugten Ausführungsform der Erfindung;
- Fig. 8:: eine erste bevorzugte Ausführungsform einer Nachbildung von Stammzellnischen eines Knochenmarks;
- Fig. 9:: eine zweite bevorzugte Ausführungsform einer Nachbildung von Stammzellnischen eines Knochenmarks;
- Fig. 10:: eine Detaildarstellung der in Fig. 9 gezeigten Nachbildung;
- Fig. 11:: eine dritte bevorzugte Ausführungsform einer Nachbildung von Stammzellnischen eines Knochenmarks;
- Fig. 12:: eine vierte bevorzugte Ausführungsform einer Nachbildung von Stammzellnischen eines Knochenmarks; und
- Fig. 13:: eine fünfte bevorzugte Ausführungsform einer Nachbildung von Stammzellnischen eines Knochenmarks in einer Anreihung.

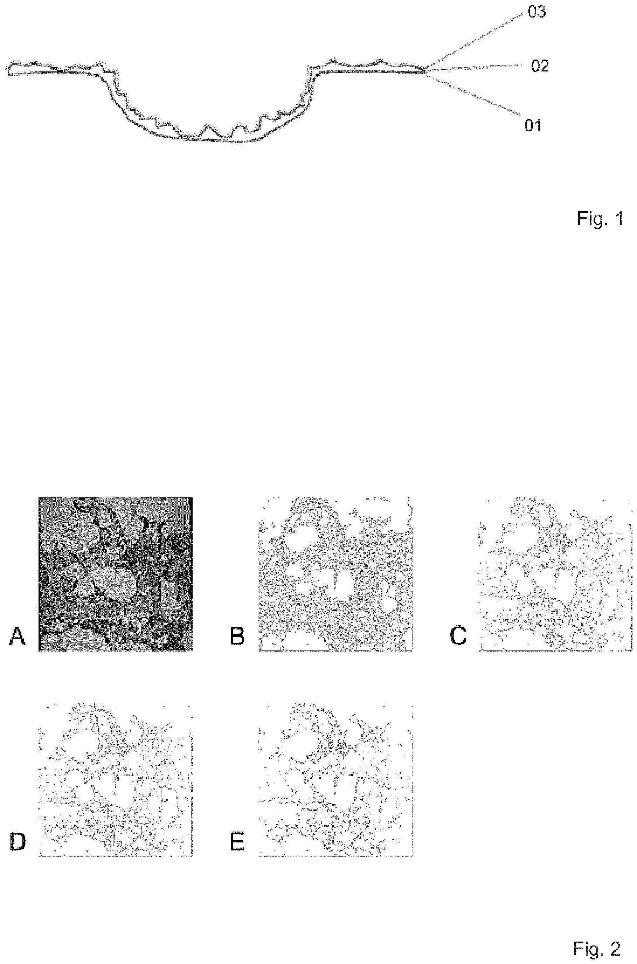

Fig. 1 zeigt das Ziel einer bevorzugten Ausführungsform des erfindungsgemäßen Verfahrens in einer Querschnittsdarstellung, nämlich der Aufbau einer hierarchischen Struktur in Form eines strukturierten Substrates, welches eine Vielzahl an Stammzellnischen eines biologischen Gewebes eines Organismus synthetisch nachbildet, bestehend aus einer groben Mesostruktur 01, einer feinen Mikrostruktur 02 und einer noch feineren Nanostruktur 03. Hierfür werden bevorzugt thermoplastische Folien mit unterschiedlichen Methoden strukturiert. Anschließend erfolgt eine Kultivierung von hämatopoietischen Stammzellen (nicht dargestellt) mit dem Ziel der Aufrechterhaltung ihres undifferenzierten Status.

Fig. 2 zeigt ein erfindungsgemäß erstelltes Originalbild A eines Knochenmarksabschnittes und daraus erfindungsgemäß erzeugte Binärbilder B, C, D, E nach Anwendung von Kantendetektor-Algorithmen. Die Binärbilder B, C, D, E stellen Strukturbilder dar, welche ausgehend von dem Originalbild A gewonnen wurden. Die Binärbilder B, C, D, E zeigen Kanten, welche Wandungen von Stammzellnischen im Knochenmark repräsentieren. Die Kanten wurden aus dem Originalbild A, welches ein Grauwert- oder RGB-Bild sein kann, mit verschiedenen Algorithmen detektiert.

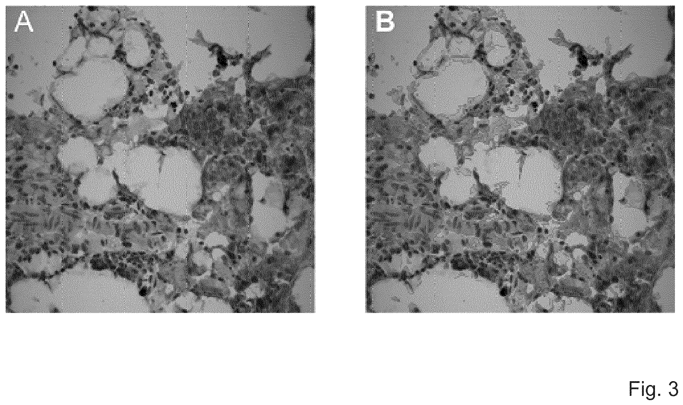

Fig. 3 zeigt einen Vergleich zwischen dem bereits in Fig. 2 gezeigten Originalbild A und einem erfindungsgemäß erstellten Binärbild nach Anwendung des Canny-Detektors. Es sind das Originalbild des Knochenmarkschnittes A und das Originalbild mit überlagerten, extrahierten Strukturen nach einer Kantendetektion mit dem Canny-Detektor B dargestellt.

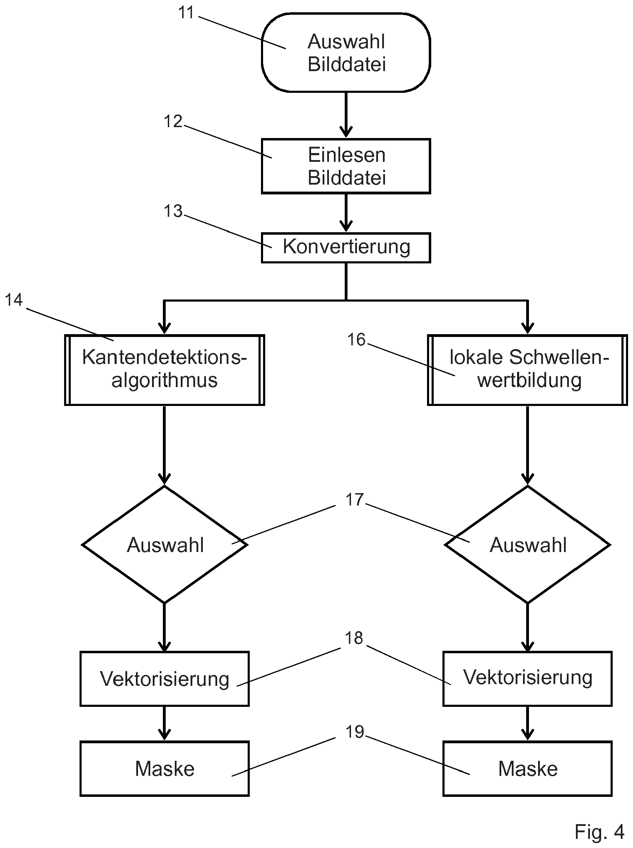

Fig. 4 zeigt ein Flussdiagramm einer Erzeugung lithographischer Masken aus Daten eines Abbildes eines mindestens eine Stammzellnische umfassenden Gewebes gemäß einer bevorzugten Ausführungsform der Erfindung. Das Flussdiagramm beschreibt den Ablauf der Extrahierung von Strukturen aus einer Bilddatei unter der Verwendung von verschiedenen Algorithmen der Binärbilderstellung und deren Verwendung im Design einer Photomaske. Nach einer Auswahl 11 einer Bilddatei und Einlesen 12 der Daten der Bilddatei in ein Computerprogramm zur Lösung mathematischer Probleme wird die Bilddatei aus dem RGB-Farbraum bzw. aus einem Grauwertbereich in ein Binärbild 13 umgewandelt. Durch Verwendung eines Kantendetektionsalgorithmus 14 und einer lokalen Schwellenwertberechnung 16 werden Strukturbilder gewonnen. Es erfolgt eine Auswahl 17 der geeignetsten Strukturbilder. Die Strukturbilder werden einer Vektorisierung 18 unterzogen, sodass Vektordaten gewonnen werden. Aus den Vektordaten werden lithographische Masken 19 erstellt.

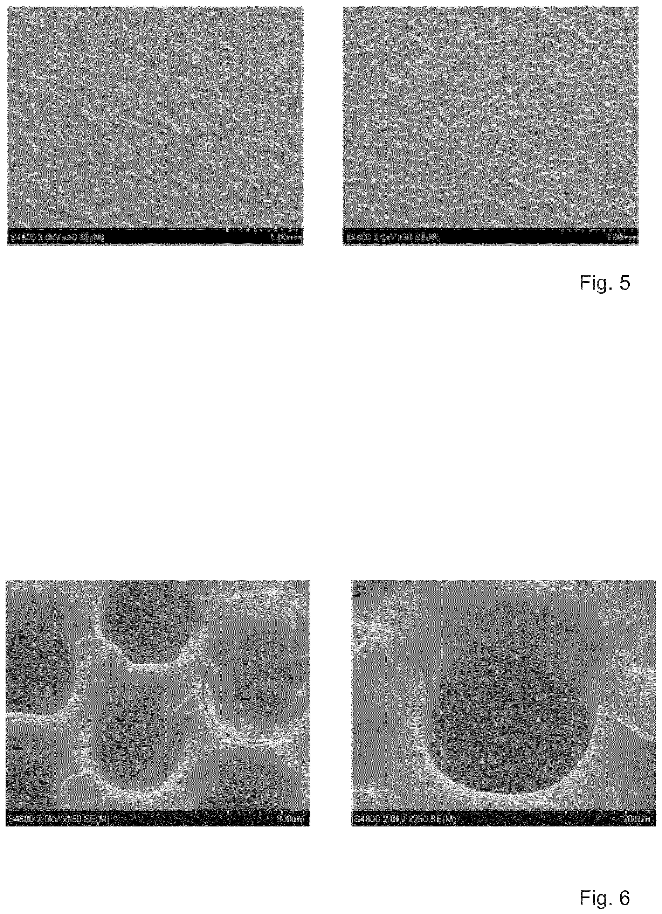

Fig. 5 zeigt Rasterelektronenmikroskopaufnahmen von erfindungsgemäß heißgeprägten Polycarbonatfolien. Durch Heißprägen wurden mit einer lithographischen Maske, die aus einer durch Abbilden und Filtern erhaltenen Knochenmarkstruktur erstellt wurde, 50 µm dicke Polycarbonatfolien mit feinen Strukturen strukturiert.

Fig. 6 zeigt weitere Rasterelektronenmikroskopaufnahmen der in Fig.5 gezeigten Polycarbonatfolien nach einem Thermoformen. Nach dem Heißprägen mit einer durch Abbilden und Filtern erhaltenen Knochenmarkstruktur wurden die 50 µm dicken Folien in einem Mikrothermoformprozess ergänzend mit einer groben Struktur strukturiert. Dafür wurden Formwerkzeuge mit Kavitäten verwendet. Die Kavitäten dieser Formwerkzeuge haben einen Durchmesser von 300 µm und eine Tiefe von ebenfalls 300 µm. Damit sind diese Strukturen aufgrund ihrer Größe denen im trabekulären Knochen sehr ähnlich. Dabei kommen die markierten Ausformungen dem Original am nächsten.

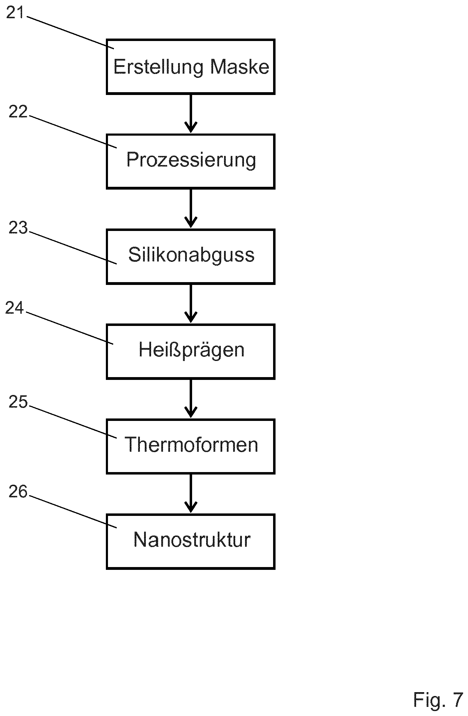

Fig. 7 zeigt ein Flussdiagramm einer physikalischen Oberflächenmodifikation gemäß einer bevorzugten Ausführungsform der Erfindung. Das Flussdiagramm zeigt den Ablauf der physikalischen Oberflächenmodifikation zum Aufbau einer hierarchischen Architektur eines erfindungsgemäß herzustellenden strukturierten Substrates. Nach der in Fig. 4 veranschaulichen Erstellung einer lithographischen Maske 21 und nach einer Prozessierung eines Wafers 22 wird ein Silikonabguss 23 eines Masters hergestellt, wodurch eine Folie resultiert. Danach erfolgt die Modifikation der Folie in einem Heißprägeschritt 24 mit einem Werkzeug, welches durch Anwenden einer lithographischen Maske erstellt wurde, die aus einer durch Abbilden und Filtern erhaltenen Struktur eines Knochenmarks erzeugt wurde. Anschließend werden in einem Mikrothermoformprozess 25 Strukturen eines trabekulären Knochens eingeprägt, wofür ein weiteres Werkzeug verwendet wird, welches durch Anwenden der lithographischen Maske erstellt wurde. In einem finalen Prozessschritt erfolgt das Aufbringen einer Nanostruktur 26 durch Dip-Coating und Inkubieren der Folie in einer Lösung oder Ätzen.

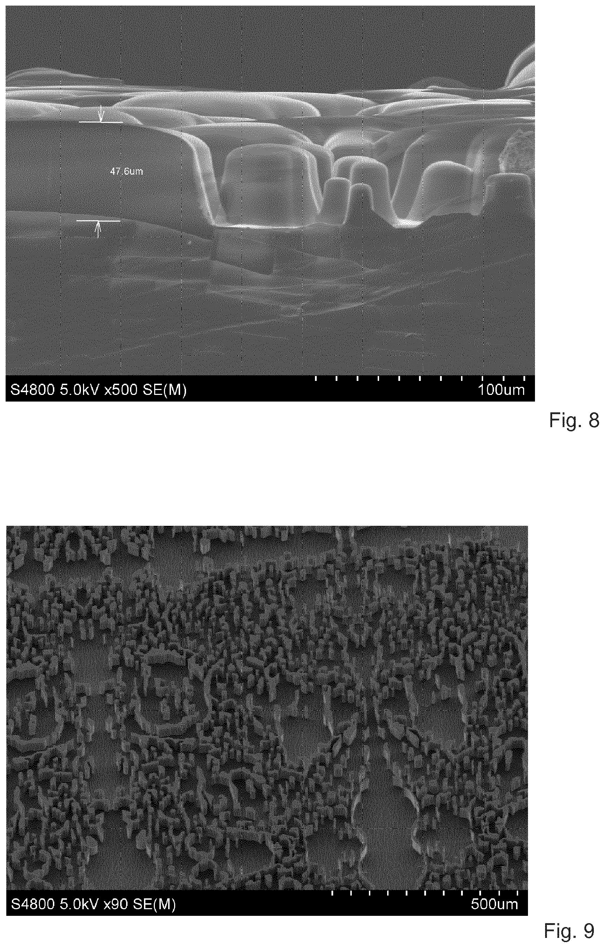

Fig. 8 zeigt eine Rasterelektronenmikroskopaufnahme einer ersten bevorzugten Ausführungsform einer Nachbildung von Stammzellnischen eines Knochenmarks. Diese Ausführungsform ist durch ein strukturiertes Substrat aus Polydimethylsiloxan gebildet, welches in einer Form abgegossen wurde.

Fig. 9 zeigt eine Rasterelektronenmikroskopaufnahme einer zweiten bevorzugten Ausführungsform einer Nachbildung von Stammzellnischen eines Knochenmarks. Diese Ausführungsform ist durch ein strukturiertes Substrat aus Silicium gebildet, welches mit einem Prägewerkzeug (nicht gezeigt) strukturiert wurde.

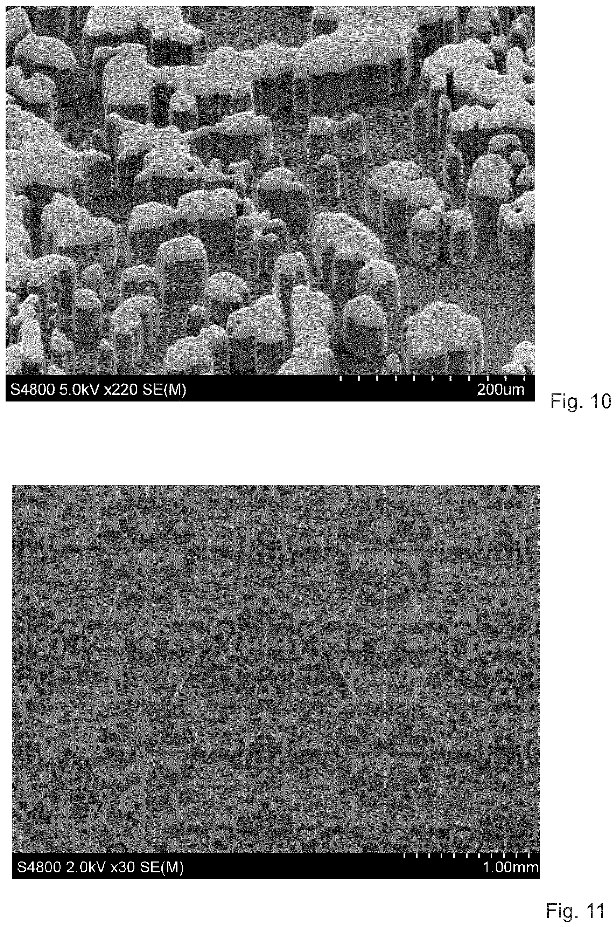

Fig. 10 zeigt eine Detaildarstellung der in Fig. 9 gezeigten Nachbildung.

Fig. 11 zeigt eine Rasterelektronenmikroskopaufnahme einer dritten bevorzugten Ausführungsform einer Nachbildung von Stammzellnischen eines Knochenmarks. Diese Ausführungsform ist durch ein strukturiertes Substrat aus Silicium gebildet, welches durch ein reaktives Ionenätzen strukturiert wurde.

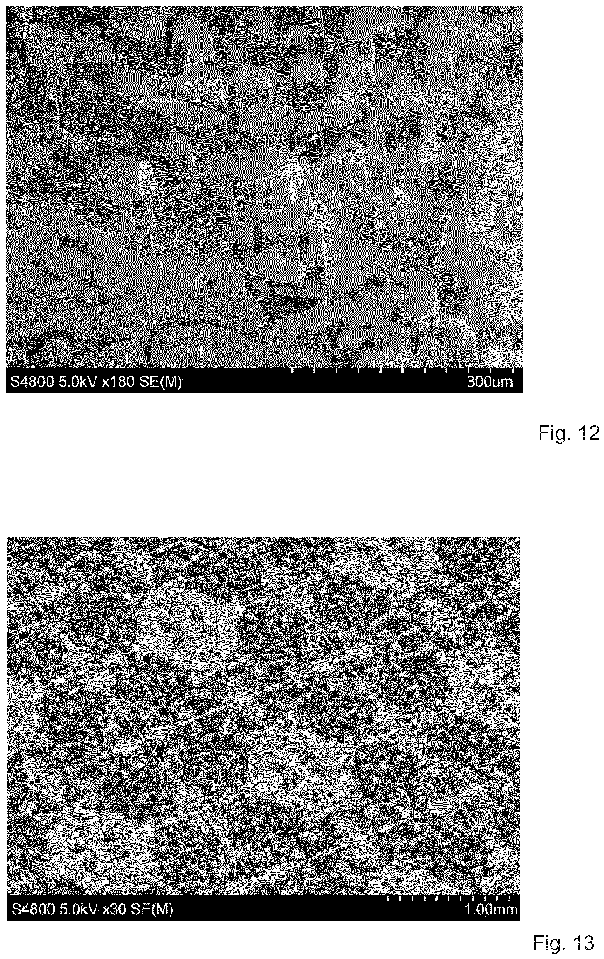

Fig. 12 zeigt eine Rasterelektronenmikroskopaufnahme einer vierten bevorzugten Ausführungsform einer Nachbildung von Stammzellnischen eines Knochenmarks. Diese Ausführungsform ist durch ein strukturiertes Substrat aus einem Borosilikatglas gebildet, bei welchem zur Strukturierung Kanten verrundet wurden.

Fig. 13 zeigt eine Rasterelektronenmikroskopaufnahme einer fünften bevorzugten Ausführungsform einer Nachbildung von Stammzellnischen eines Knochenmarks. Bei dieser Ausführungsform ist eine extrahierte Struktur zur großflächigen Reproduktion mehrfach aneinandergereiht.

### Bezugszeichenliste

- 01: Mesostruktur
- 02: Mikrostruktur
- 03: Nanostruktur
- 04: -
- 05: -
- 06: -
- 07: -
- 08: -
- 09: -
- 10: -
- 11: Auswahl einer Bilddatei
- 12: Einlesen der Daten der Bilddatei
- 13: Binärbild
- 14: Kantendetektionsalgorithmus
- 15: -
- 16: lokale Schwellenwertberechnung
- 17: Auswahl der geeignetsten Strukturbilder
- 18: Vektorisierung
- 19: lithographische Masken
- 20: -
- 21: Erstellung einer lithographischen Maske
- 22: Prozessierung eines Wafers
- 23: Silikonabguss
- 24: Heißprägeschritt
- 25: Mikrothermoformprozess
- 26: Aufbringen einer Nanostruktur

## Patentansprüche

1. Verfahren zum Nachbilden einer Stammzellnische eines Organismus, folgende Schritte umfassend:
- Erzeugen eines Abbildes eines mindestens eine Stammzellnische umfassenden Gewebes eines Organismus;
- Filtern (16, 17) des Abbildes, um ein Strukturbild der abgebildeten Stammzellennische zu erzeugen;
- Erzeugen (21) einer lithographischen Maske (19) aus dem Strukturbild; und
- Strukturieren (22, 23, 24, 25, 26) eines Ausgangsmaterials eines Substrates durch Anwenden der lithographischen Maske (19), wodurch ein strukturiertes Substrat erhalten wird, welches die Nachbildung der abgebildeten Stammzellnische des Organismus darstellt.

2. Verfahren nach Anspruch 1, **dadurch gekennzeichnet, dass** das Strukturbild ein Teilstrukturbild für grobe Strukturen der abgebildeten Stammzellnische und ein Teilstrukturbild für feine Strukturen der abgebildeten Stammzellnische umfasst; wobei die lithographische Maske (19) eine Teilmaske für grobe Strukturen umfasst, die aus dem Teilstrukturbild für grobe Strukturen erzeugt wird; und wobei die lithographische Maske (19) eine Teilmaske für feine Strukturen umfasst, die aus dem Teilstrukturbild für feine Strukturen erzeugt wird.

3. Verfahren nach Anspruch 2, **dadurch gekennzeichnet, dass** die feinen Strukturen eine maximale Elementgröße zwischen 50 µm und 75 µm aufweisen, und dass die groben Strukturen eine minimale Elementgröße zwischen 50 µm und 75 µm aufweisen.

4. Verfahren nach Anspruch 2 oder 3, **dadurch gekennzeichnet, dass** das Filtern (16, 17) des Abbildes verschiedene Kantenanalysen umfasst, mit denen das Teilstrukturbild für die groben Strukturen und das Teilstrukturbild für die feinen Strukturen bestimmt werden.

5. Verfahren nach einem der Ansprüche 1 bis 4, **dadurch gekennzeichnet, dass** mit der lithographischen Maske (19) zunächst ein Werkzeug zum Verformen des Ausgangsmaterials des Substrates erstellt wird, wobei anschließend das Ausgangsmaterial des Substrates mit dem Werkzeug strukturiert wird.

6. Verfahren nach dem auf Anspruch 2 rückbezogenen Anspruch 5, **dadurch gekennzeichnet, dass** das Werkzeug zum Verformen des Ausgangsmaterials ein Werkzeug zur Erzeugung grober Strukturen und ein Werkzeug zur Erzeugung feiner Strukturen umfasst, wobei das Werkzeug zur Erzeugung grober Strukturen mit der Teilmaske für grobe Strukturen erstellt wird, und wobei das Werkzeug zur Erzeugung feiner Strukturen mit der Teilmaske für feine Strukturen erstellt wird.

7. Verfahren nach Anspruch 6, **dadurch gekennzeichnet, dass** das Werkzeug zur Erzeugung feiner Strukturen durch ein Prägewerkzeug zum Heißprägen oder durch ein Prägewerkzeug für eine Nanoprägelithografie gebildet ist, und dass das Werkzeug zur Erzeugung grober Strukturen durch ein Thermoformwerkzeug gebildet ist.

8. Verfahren nach einem der Ansprüche 1 bis 7, **dadurch gekennzeichnet, dass** das strukturierte Substrat mit mindestens einer Stammzelle besiedelt wird.

## Claims

1. Method for reproducing a stem cell niche of an organism, comprising the following steps:
- Creation of an image of a tissue comprising at least one stem cell niche of an organism;
- Filtering (16, 17) of the image in order to produce a structural pattern of the reproduced stem cell niche;
- Creation (21) of a lithographic mask (19) from the structural pattern; and
- Structuring (22, 23, 24, 25, 26) of a starting material of a substrate through application of the lithographic mask (19), whereby a structured substrate is obtained which represents the reproduction of the imaged stem cell niche of the organism.

2. Method according to Claim 1, **characterized in that** the structural pattern comprises a substructural pattern for coarse structures of the imaged stem cell niche and a substructural pattern for fine structures of the imaged stem cell niche; wherein the lithographic mask (19) comprises a submask for coarse structures that is produced from the substructural pattern for coarse structures; and wherein the lithographic mask (19) comprises a submask for fine structures that is produced from the substructural pattern for fine structures.

3. Method according to Claim 2, **characterized in that** the fine structures have a maximum feature size of between 50 µm and 75 µm, and that the coarse structures have a minimum feature size of between 50 µm and 75 µm.

4. Method according to Claim 2 or 3, **characterized in that** the filtering (16, 17) of the image comprises different edge analyses, with which the substructural pattern for the coarse structures and the substructural pattern for the fine structures are determined.

5. Method according to one of claims 1 to 4, **characterized in that** a tool is first created with the lithographic mask (19) for deforming the starting material of the substrate, after which the starting material of the substrate is structured with the tool.

6. Method according to Claim 5 which refers back to Claim 2, **characterized in that** the tool for deforming the starting material preferably includes a tool for creating coarse structures and a tool for creating fine structures, with the tool for creating coarse structures being created with the submask for coarse structures, and with the tool for creating fine structures being created with the submask for fine structures.

7. Method according to Claim 6, **characterized in that** the tool for creating fine structures is constituted by an embossing die for hot-embossing or by an embossing die for nanoimprint lithography, and that the tool for creating coarse structures is constituted by a thermoforming mold.

8. Method according to one of Claims 1 to 7, **characterized in that** the structured substrate is populated with at least one stem cell.

## Revendications

1. Procédé de reproduction d'une niche de cellules souches d'un organisme, comprenant les étapes suivantes :
- créer une représentation d'un tissu appartenant à un organisme et comprenant au moins une niche de cellules souches ;
- filtrer (16, 17) ladite représentation afin de créer une image structurelle de la niche de cellules souches représentée ;
- créer (21) un masque de lithographie (19) à partir de ladite image structurelle; et
- structurer (22, 23, 24, 25, 26) un matériau de départ d'un substrat par application du masque de lithographie (19), pour ainsi obtenir un substrat structuré lequel correspond à la reproduction de la niche de cellules souches représentée appartenant audit organisme.

2. Procédé selon la revendication 1, **caractérisé en ce que** ladite image structurelle comprend une image structurelle partielle destinée aux structures grossières de la niche de cellules souches représentée et une image structurelle partielle destinée aux structures fines de la niche de cellules souches représentée ; le masque de lithographie (19) comprenant un masque partiel qui est destiné aux structures grossières et qui est créé à partir de l'image structurelle partielle destinée aux structures grossières ; et le masque de lithographie (19) comprenant un masque partiel qui est destiné aux structures fines et qui est créé à partir de l'image structurelle partielle destinée aux structures fines.

3. Procédé selon la revendication 2, **caractérisé en ce que** lesdites structures fines présentent une taille d'éléments maximale comprise entre 50 µm et 75 µm, et que les structures grossières présentent une taille d'éléments minimale comprise entre 50 µm et 75 µm.

4. Procédé selon les revendications 2 ou 3, **caractérisé en ce que** le filtrage (16, 17) de la représentation comprend différentes analyses des contours permettant de définir l'image structurelle partielle destinée aux structures grossières et l'image structurelle partielle destinée aux structures fines.

5. Procédé selon l'une des revendications 1 à 4, **caractérisé en ce que** le masque de lithographie (19) permet d'obtenir d'abord un outil destiné à déformer le matériau de départ du substrat, pour ensuite structurer le matériau de départ du substrat à l'aide dudit outil.

6. Procédé selon la revendication 5 se référant à la revendication 2, **caractérisé en ce que** l'outil destiné à déformer le matériau de départ comprend un outil qui permet de créer des structures grossières et un outil qui permet de créer des structures fines, l'outil qui permet de créer des structures grossières étant obtenu à l'aide du masque partiel qui est destiné aux structures grossières, et l'outil qui permet de créer des structures fines étant obtenu à l'aide du masque partiel qui est destiné aux structures fines.

7. Procédé selon la revendication 6, **caractérisé en ce que** l'outil qui permet de créer des structures fines est formé par un outil de marquage destiné réaliser à un marquage à chaud, ou par un outil de marquage destiné à réaliser un marquage lithographique à l'échelle nano, et que l'outil qui permet de créer des structures grossières est formé par une outil de thermoformage.

8. Procédé selon l'une des revendications 1 à 7, **caractérisé en ce que** le substrat structuré est soumis à une colonisation par au moins une cellule souche.
